# EUROPEAN PATENT SPECIFICATION

(11) **EP 1 437 140 B1**
(45) Date of publication and mention of the grant of the patent: **15.04.2009**
(21) Application number: 02754173.9
(22) Date of filing: 09.09.2002
(51) Int. Cl.: A61K 33/44, A61P 5/16

(54) **ORAL PHARMACEUTICAL FORMULATION CONTAINING ACTIVE CARBON AND USE OF THE SAME**
ORALE PHARMAZEUTISCHE FORMULIERUNG MIT AKTIVKOHLE UND IHRE VERWENDUNG
FORMULATION PHARMACEUTIQUE ORALE CONTENANT DU CHARBON ACTIF ET UTILISATION DE CETTE FORMULATION

(30) Priority: 13.09.2001 CN 1128840X
(43) Date of publication of application: 14.07.2004
(73) Proprietor: Chen, Decai, Sichuan 610000 (CN)
(72) Inventor: Chen, Decai, Sichuan 610000 (CN)
(74) Representative: Schnabel, Jörg
(86) International application number: PCT/CN2002/000630
(87) International publication number: WO 2003/022292

(56) References cited:
- HACK JASON B; LEVISS JOHN A; NELSON LEWIS S; HOFFMAN ROBERT S: "Severe symptoms following a massive intentional L-thyroxine ingestion" VETERINARY AND HUMAN TOXICOLOGY, vol. 41, no. 5, October 1999 (1999-10), pages 323-326, XP008046262
- COHEN J H III; INGBAR S H; BRAVERMAN L E: "THYROTOXICOSIS DUE TO INGESTION OF EXCESS THYROID HORMONE" ENDOCRINE REVIEWS, vol. 10, no. 2, 1989, pages 113-124, XP008046261
- MANDEL S H; MAGNUSSON A R; BURTON B T; SWANSON J R; LAFRANCHI S H: "Massive levothyroxine ingestion. Conservative management" CLINICAL PEDIATRICS, vol. 28, no. 8, August 1989 (1989-08), pages 374-376, XP008046266
- HERRMANN J; RUDORFF K H; GOCKENJAN G, KONIGSHAUSEN TH, GRABENSEE B, KRUSKEMPER H L: "Charcoal haemoperfusion in thyroid storm" THE LANCET, vol. 309, no. 8005, 1977, page 248, XP008046264
- CANDRINA R; DI STEFANO O; SPANDRIO S; GIUSTINA G: "TREATMENT OF THYROTOXIC STORM BY CHARCOAL PLASMA PERFUSION" JOURNAL OF ENDOCRINOLOGICAL INVESTIGATION, vol. 12, no. 2, 1989, pages 133-134, XP008046263
- BINIMELIS J; BASSAS L; MARRUECOS L; RODRIGUEZ J; DOMINGO M L; MADOZ P; ARMENGOL S; MANGUES M A; DE LEIVA A: "Massive thyroxine intoxication: evaluation of plasma extraction" INTENSIVE CARE MEDICINE, vol. 13, no. 1, 1987, pages 33-38, XP008046260
- 'Clinical progress of active carbon in treating hyperlipemia' XI BEI YAO XUE ZA ZHI vol. 12, no. 2, April 1997, page 91
- 'The application of active carbon microparticales (CH40) in cervical lymph node dissection for oral saquamous cell carcinoma' ZHONG GUO YI KE DA XUE XUE BAO vol. 28, no. 3, June 1999, pages 215 - 216
- 'Use of active carbon microparticales in treating carcinoma ventriculi' GUO WAI YI XUE ZHONG LIU XUE FEN CE vol. 265, no. 5, October 1999, pages 298 - 300

## Description

### Field of the Invention

This invention relates to an oral drug preparation and its usage, especially using active carbon in oral drug preparation and its application in hyperthyroidism therapy.

### Background of the Invention

Hyperthyroidism is a set of familiar clinical syndrome. Survey data have showed the prevalence rate of the disease is between 0.5% and 1.5%. Hyperthyroidism has become one of the most dominant diseases of out-patient service and hospitalization in department of endocrinology, nuclear medicine and correlative departments, while the incidence rate of which is up-trend obviously. Its main manifestation is elevated thyroid hormones in serum, which results in a series of clinical symptoms of increased metabolism, for instance, easy hungry, profuse sweating, emaciation and diarrhea. Because the disease makes the sufferer in a elevated metabolism situation, a list of serious complication will appear if the patient's condition can not be improved in time such as cardiopathy and myopathy of hyperthyroidism, osteoporosis, electrolyte disturbance, and so on. So, medical society has attached much importance to the disease. But there has little hypostatic breakthrough on the cognition of the mechanism of Graves' Disease-a main cause of hyperthyroidism, and also, the methods of treatment stay around that of 1970s'. There are some shortcomings in current treatment steps, which can be sum up as follows: (1) antithyroid drugs. This method displays its effect slowly. Although given sufficiency antithyroid drugs, Patients may spend 4 to 8 weeks or longer to acquire normal level of serum thyroid hormones and remission of clinical symptoms. In the initial stage of drug therapy, physicians must observe side effects carefully after the patients taking medicine because of the rather bigger dosage of medication, for example, decrease of Granulocytic cells, allergic reaction, hepatic disfunction. It's not a convenient way for out-patient. Patients accepting care in hospitalization are not willing to discharge if the symptoms are not controlled completely. If the patients discharge after fully remission of hyperthyroidism symptoms, the inpatient period will be prolonged significantly, with medical cost increased and medical resources wasted greatly. Patients of serious hyperthyroidism, cardiopathy of hyperthyroidism or crisis of hyperthyroidism have danger of death at any time, because this method of therapy has slow effect and can not improve patients' condition promptly and effectively. For patients in gestation or lactation, patients with markedly decreased granulocytic cells or hypersusceptibility to antithyroid drugs, this step is unsuitable, either. All of these conditions are not rare in clinic. (2) Radioactive iodine¹³¹ isotope or surgery. Serum thyroid hormones must be decreased to normal or near normal before these kinds of steps. It will cost the patient a long time using antithyroid medication to prepare for radioactive iodine¹³¹ isotope or surgery therapy. After putting these kinds of treatment into practice, serum thyroid hormones will increase obviously followed by aggravation of hyperthyroidism manifestation because a great deal of thyroid follicles were destroyed or the thyroid gland was irritated. More active measures need to be adopted to depress serum thyroid hormones rapidly under these circumstances. But antithyroid drugs could not achieve anticipant results during a short period. There exits many idiographic difficulties. Patients in gestation or lactation, patients with serious cardiopathy of hyperthyroidism or markedly decreased granulocytic cells as well as those in crisis of hyperthyroidism could not accept the two measures as above-mentioned (see in thyroid gland diseases, page 92-104, edited by Xuwen Gao and Jilian Li. Publishing house of People's public health. 1999).

### Summary of the Invention

The invention is exactly for the sake of overcoming the deficiency of current techniques above-mentioned and provides a new oral drug preparation of activated charcoal. This oral drug preparation could mainly combine thyroid hormones excreting to intestinal tract, block the enterohepatic circulation of thyroid hormones, which will result in rapid decrease of serum thyroid hormones level in hyperthyroidism patients.

Oral drug preparation using this invention for hyperthyroidism treatment contains activated charcoal and necessary accessories or acceptable carriers in pharmacy, in which content of activated charcoal (according to weight percent) is at least 15%. Contents of other accessories contain dextrin starch, starch plasm, peach resin, peach resin plasm and magnesium stearate, which may be changed according to demand of preparation. This drug preparation may be composed of pure activated charcoal completely and was used directly. It can be made in tablet or capsule. The action principle of this preparation is as follows: Hyperthyroidism is developed by an elevated serum thyroid hormones status. To treat hyperthyroidism successfully, we should firstly depress serum thyroid hormones level to normal. Under normal circumstance, thyroid hormones were composed by thyroid gland, stored in thyroid follicles, and released into blood circulation by thyroid follicles. Thyroid hormones in blood include two parts, those combined to protein and free (not combined to protein). Free thyroid hormones have weak physiological function. After affected by liver deiodinase, about two third free thyroid hormones change into triiodotyrosine with strong activity, which will reenter blood and bring its physiological function into full play. Approximately one third free thyroid hormones combine directly to glucuronic acid or sulfate salt without deiodination and excrete into small intestine with bile, half of which will be reabsorbed into blood. That is the enterohepatic circulation of thyroid hormones. Those not reabsorbed by intestine will be excreted with feces. Free thyroid hormones level increases obviously in hyperthyroidism patients. Also, ratio of thyroid hormones excreted into intestine by liver and those taking part in enterohepatic circulation will increase markedly. If thyroid hormones excreted into intestine where combined by special drug, the ejectment of thyroid hormones by intestine tract will increase, and then the enterohepatic circulation of thyroid hormones will be blocked and depress serum thyroid hormones level rapidly. This special drug is just activated charcoal, which is called medical charcoal when it reaches standards of medication usage. It is in a particle form not decomposed in human's intestinal tract and can not be absorbed into blood while achieving effects as mentioned above

In addition, activated charcoal can bind or adsorb iodine - a necessary material for thyroid hormones synthesis, further depressing thyroid hormones production. Meanwhile, it can suppress or adsorb bacteria in enteron such as Yersinia enterocolitica which could evoke hyperthyroidism. In this way, activated charcoal has effects on three approaches: reduce synthesis material of thyroid hormones, eliminate bacteria evoking hyperthyroidism and decrease formed thyroid hormones. As a result, it can depress serum thyroid hormones level significantly in a short time.

Pharmacodynamics trial using this activated charcoal oral preparation in mice was carried through. Mice were fed with a dosage of 1g·d⁻¹·kg⁻¹ for a successive week and the anti-anoxia ability of mice increased obviously.

Pharmacodynamics trial using this activated charcoal oral preparation in Rats was carried through. Rats were fed with a dosage of 1g·d⁻¹·kg⁻¹ for two successive weeks and serum thyroid hormones level of rats decreased significantly.

Activated charcoal is not absorbed into blood and it works only in intestinal tract. There aren't any side effects of common antithyroid drugs, while it has the effect on depressing thyroid hormones level So, activated charcoal oral preparation is available absolutely in all kinds of clinical conditions contraindicating of antithyroid drugs, such as hypersusceptibility, granulocytopenia or deficiency of granulocyte, gestation and lactation: It is also applicable when antithyroid drugs can not work promptly, such as in the early days of general hyperthyroidism treatment, serious hyperthyroidism, hyperthyroidism crisis, cardiopathy of hyperthyroidism, preoperative preparation and postoperative treatment of iodine¹³¹ isotope or surgery therapy. The clinical prospective investigation of more than 80 cases in about one year indicated that the rate of decrease in serum thyroid hormones (TT₃, TT₄, FT₃, FT₄) level of patients in trial group accepting activated charcoal with a dosage of 1g to 150g together with routine antithyroid drugs was higher than those in control group (discrepancy nearly 2 times) significantly, and the speed of increase of serum TSH of patients in trial group was higher than those in control group (discrepancy nearly 5 times) significantly.

Compared with existing treatment technology, this invention has virtues as follows: (1) Nice safety and extensive applicable range. It is not restricted in patients with gestation and granulocyte deficiency. It avoids various kinds of side effects in existing antithyroid drugs and is available in all hyperthyroidism patients. (2) Curative effects appear rapidly and certainly. Decrease of thyroid hormones level and remission of clinical symptoms are more quickly, which proved the effectiveness of activated charcoal oral preparation in hyperthyroidism treatment. (3) Managing and convenient. The cost price of activated charcoal is cheap and it is convenient to take the medicine. If combined to other antithyroid drugs, it can control hyperthyroidism faster, which will alleviate suffering of hyperthyroidism patients, shorten the hospitalization period and save medical costs. (4) Without obvious side effects. Before and after taking this oral medicine, the blood routine examination, hepatic function, renal function, blood lipid, urine and stool routine examination of the patients don't change. There isn't other discomfort in clinical observation.

### Detailed Descriptioin of the Preferred Embodiment

Hereinafter, this invention will be narrated in details using preferred embodiments putting into practice:
The extraordinary item of oral drug preparation using activated charcoal for hyperthyroidism therapy is giving priority to activated charcoal and adding necessary accessories in preparation of oral drug for hyperthyroidism therapy. The prescription of activated charcoal oral drug according to weight percent may be composed of activated charcoal 30% to 80%, dextrin 4% to 15%, starch 4% to 15%, starch plasm 4% to 15%, peach resin 4% to 15%, peach resin plasm 3% to 7% and magnesium stearate 1% to 3%

Example 1. According to 100g activated charcoal oral drug preparation, tablet is made by a prescription of activated charcoal 80g, dextrin 4g, starch 4g, starch plasm 4g, peach resin 4g, peach resin plasm 3g and magnesium stearate 1g. There is no obvious side effect when patients take oral drug preparation containing activated charcoal in a dosage of 1g to 150g per day Serum thyroid hormones level (TT₃, TT₄, FT₃, FT₄) is near to normal on the whole.

Example 2. According to 100g activated charcoal oral drug preparation, the prescription is composed of activated charcoal 56g, dextrin 7g, starch 7g, starch plasm 6g, peach resin 6g, peach resin plasm 17g and magnesium stearate 1g. There is no obvious side effect when patients take oral drug preparation containing activated charcoal in a dosage as in example 1. The serum thyroid hormones level (TT₃, TT₄, FT₃, FT₄) decrease obviously.

Example 3. According to 100g activated charcoal oral drug preparation, the prescription is composed of activated charcoal 30g, dextrin 15g, starch 15g, starch plasm 15g, peach resin 15g, peach resin plasm 7g and magnesium stearate 3g. There is no obvious side effect when patients take oral drug preparation containing activated charcoal in a dosage as in example 1. Serum thyroid hormones level (TT₃, TT₄, FT₃, FT₄) is near to normal on the whole.

Example 4. The extraordinary item of oral drug preparation using activated charcoal for hyperthyroidism therapy is to apply activated charcoal into oral drug preparation for hyperthyroidism therapy. According to 1 00g activated charcoal oral drug preparation, the prescription is composed of activated charcoal 19g, dextrin 10g, starch 10g, starch plasm 10g, peach resin 10g, peach resin plasm 40g and magnesium stearate 1g Patients take oral drug preparation containing activated charcoal in a dosage as in example 1, combining with other antithyroid drugs. Serum thyroid hormones level (TT₃, TT₄, FT₃, FT₄) decreases rapidly,

EXample 5 The activated charcoal oral drug preparation may use activated charcoal as raw material completely and apply directly. There is no side effect when patients took 150g per day. Serum thyroid hormones level (TT₃, TT₄, FT₃, FT₄) is near to normal on the whole.

Example 6. The extraordinary item of oral drug preparation using activated charcoal for hperthyroidism therapy is to apply activated charcoal into oral drug preparation for hyperthyroidism therapy. The activated charcoal oral preparation may use activated charcoal in sell as long as the dosage of which is in the range of this invention.

## Claims

1. Use of activated charcoal for the production of an oral drug preparation for hyperthyroidism therapy.

2. The use according to claim 1, wherein the oral drug preparation is a tablet or capsule.

3. The use according to claim 1 or 2, wherein the activated charcoal oral drug is composed of 100% activated charcoal.

4. The use according to claim 1 or 2, wherein the activated charcoal oral drug is composed of activated charcoal 80% by weight, dextrin 4% by weight, starch 4% by weight, starch plasm 4% by weight, peach resin 4% by weight, peach resin plasm 3% by weight and magnesium stearate 1 % by weight.

5. The use according to claim 1 or 2, wherein the activated charcoal oral drug is composed of activated charcoal 30% by weight, dextrin 15% by weight, starch 15% by weight, peach resin 15% by weight, peach resin plasm 7% by weight, magnesium stearate 3% by weight and 15% by weight starch plasm.

6. The use according to claim 1 or 2, wherein the activated charcoal oral drug is composed of activated charcoal 56% by weight, dextrin 7% by weight, starch 7% by weight, peach resin 6% by weight, peach resin plasm 17% by weight, magnesium stearate 1% by weight and 6% by weight starch plasm.

7. The use according to claim 1 or 2, wherein the activated charcoal oral drug is composed of activated charcoal 19% by weight, dextrin 10% by weight, starch 10% by weight, peach resin 10% by weight, peach resin plasm 40% by weight, magnesium stearate 1% by weight and 10% by weight starch plasm.

## Patentansprüche

1. Verwendung von Aktivkohle zur Herstellung eines oralen Arzneimittelpräparats zur Behandlung von Hyperthyreose.

2. Verwendung nach Anspruch 1, wobei das orale Arzneimittelpräparat eine Tablette oder Kapsel ist.

3. Verwendung nach Anspruch 1 oder 2, wobei das orale Aktivkohlearzneimittel aus 100 % Aktivkohle besteht.

4. Verwendung nach Anspruch 1 oder 2, wobei das orale Aktivkohlearzneimittel aus 80 Gew.-% Aktivkohle, 4 Gew.-% Dextrin, 4 Gew.-% Stärke, 4 Gew.-% Stärkeplasma, 4 Gew.-% Pfirsichharz, 3 Gew.-% Pfirsichharzplasma und 1 Gew.-% Magnesiumstearat besteht.

5. Verwendung nach Anspruch 1 oder 2, wobei das orale Aktivkohlearzneimittel aus 30 Gew.-% Aktivkohle, 15 Gew.-% Dextrin, 15 Gew.-% Stärke, 15 Gew.-% Pfirsichharz, 7 Gew.-% Pfirsichharzplasma, 3 Gew.-% Magnesiumstearat und 15 Gew.-% Stärkeplasma besteht.

6. Verwendung nach Anspruch 1 oder 2, wobei das orale Aktivkohlearzneimittel aus 56 Gew.-% Aktivkohle, 7 Gew.-% Dextrin, 7 Gew.-% Stärke, 6 Gew.-% Pfirsichharz, 17 Gew.-% Pfirsichharzplasma, 1 Gew.-% Magnesiumstearat und 6 Gew.-% Stärkeplasma besteht.

7. Verwendung nach Anspruch 1 oder 2, wobei das orale Aktivkohlearzneimittel aus 19 Gew.-% Aktivkohle, 10 Gew.-% Dextrin, 10 Gew.-% Stärke, 10 Gew.-% Pfirsichharz, 40 Gew.-% Pfirsichharzplasma, 1 Gew.-% Magnesiumstearat und 10 Gew.-% Stärkeplasma besteht

## Revendications

1. - Utilisation de charbon actif pour la production d'une préparation de médicament oral pour la thérapie de l'hyperthyroïdie.

2. - Utilisation selon la revendication 1, dans laquelle la préparation de médicament oral est un comprimé ou une capsule.

3. - Utilisation selon l'une des revendications 1 ou 2, dans laquelle le médicament oral à charbon actif est composé de 100 % de charbon actif.

4. - Utilisation selon l'une des revendications 1 ou 2, dans laquelle le médicament oral à charbon actif est composé de 80 % en poids de charbon actif, de 4 % en poids de dextrine, de 4 % en poids d'amidon, de 4 % en poids de plasma d'amidon, de 4 % en poids de résine de pêche, de 3 % en poids de plasma de résine de pêche et de 1 % en poids de stéarate de magnésium.

5. - Utilisation selon l'une des revendications 1 ou 2, dans laquelle le médicament oral à charbon actif est composé de 30 % en poids de charbon actif, de 15 % en poids de dextrine, de 15 % en poids d'amidon, de 15 % en poids de résine de pêche, de 7 % en poids de plasma de résine de pêche, de 3 % en poids de stéarate de magnésium et de 15 % en poids de plasma d'amidon.

6. - Utilisation selon l'une des revendications 1 ou 2, dans laquelle le médicament oral à charbon actif est composé de 56 % en poids de charbon actif, de 7 % en poids de dextrine, de 7 % en poids d'amidon, de 6 % en poids de résine de pêche, de 17 % en poids de plasma de résine de pêche, de 1 % en poids de stéarate de magnésium et de 6 % en poids de plasma d'amidon.

7. - Utilisation selon l'une des revendications 1 ou 2, dans laquelle le médicament oral à charbon actif est composé de 19 % en poids de charbon actif, de 10 % en poids de dextrine, de 10 % en poids d'amidon, de 10 % en poids de résine de pêche, de 40 % en poids de plasma de résine de pêche, de 1 % en poids de stéarate de magnésium et de 10 % en poids de plasma d'amidon.
